(19) Europäisches Patentamt
European Patent Office
Office européen des brevets

(11) EP 0 595 183 B1

(12) EUROPEAN PATENT SPECIFICATION

(45) Date of publication and mention of the grant of the patent:
**25.08.1999 Bulletin 1999/34**

(21) Application number: **93116993.2**

(22) Date of filing: **20.10.1993**

(51) Int Cl.[6]: **C07D 491/107**, C07D 495/10, A61K 31/415
// (C07D491/107, 311:00, 235:00), (C07D495/10, 335:00, 235:00)

(54) **Hydantoin derivatives, salts thereof and maillard reaction inhibitors comprising the same**

Hydantoinderivate, deren Salze und diese enthaltende Maillard-Reaktionsinhibitoren

Dérivés de l'hydantoine, leurs sels et inhibiteurs de la réaction maillard les contenant

(84) Designated Contracting States:
**BE CH DE FR GB IT LI**

(30) Priority: **27.10.1992 JP 28865092**

(43) Date of publication of application:
**04.05.1994 Bulletin 1994/18**

(73) Proprietor: **SANWA KAGAKU KENKYUSHO CO., LTD.**
**Nagoya, Aichi-ken (JP)**

(72) Inventors:
- **Kurono, Masayasu, c/o Sanwa Kagaku Higashi-ku, Nagoya, Aichi-ken (JP)**
- **Baba, Yutaka, c/o Sanwa Kagaku Higashi-ku, Nagoya, Aichi-ken (JP)**
- **Yamaguchi, Takuji, c/o Sanwa Kagaku Higashi-ku, Nagoya, Aichi-ken (JP)**
- **Unno, Ryoichi, c/o Sanwa Kagaku Higashi-ku, Nagoya, Aichi-ken (JP)**
- **Kimura, Hiromoto, c/o Sanwa Kagaku Higashi-ku, Nagoya, Aichi-ken (JP)**
- **Inagaki, Hideaki, c/o Sanwa Kagaku Higashi-ku, Nagoya, Aichi-ken (JP)**
- **Tomiya, Noboru, c/o Sanwa Kagaku Higashi-ku, Nagoya, Aichi-ken (JP)**
- **Suzuki, Takeshi, c/o Sanwa Kagaku Higashi-ku, Nagoya, Aichi-ken (JP)**
- **Ban, Masatoshi, c/o Sanwa Kagaku Higashi-ku, Nagoya, Aichi-ken (JP)**
- **Hibi, Chihiro, c/o Sanwa Kagaku Higashi-ku, Nagoya, Aichi-ken (JP)**
- **Sawai, Kiichi, c/o Sanwa Kagaku Higashi-ku, Nagoya, Aichi-ken (JP)**

(74) Representative:
**Wächtershäuser, Günter, Prof. Dr.**
**Patentanwalt,**
**Tal 29**
**80331 München (DE)**

(56) References cited:
**EP-A- 0 418 834**

- **CHEMICAL ABSTRACTS, vol. 117, no. 20, 16 November 1992, Columbus, Ohio, US; abstract no. 198526b, M. KURONO ET AL 'Pharmaceutical compositions containing hydantoin derivatives for treatment of blood circulation disorders' page 445 ; & JP-A-04 145 023 (SANWA KAGAKU KENKYUSHO) 19 May 1992**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to hydantoin derivatives, salts thereof, and the use of hydantoin derivatives for the manufacture of a medicament for a treatment wherein a Maillard reaction is inhibited.

In recent years, Maillard reaction of proteins in living body due to nonenzymatic glycosylation has been remarked with great interests, as one of causes on various diseases due to diabetes, arteriosclerosis and age-associated disease due to senile deterioration.

In case of the protein, the Maillard reaction firstly begins by a reaction of a reducing sugar with amino radical of the protein to form an amadori rearrangement product. When the reaction further proceeds in an advanced state, a cross-linking polymerized compound is formed (In general, such a compound has been called as "advanced glycosylation end products", and hereinafter referred abbreviately to --AGE--). The AGE is a substance with yellowish brown color, which fluoresces and has a property of binding to the protein near thereto to form a cross-linking therewith. It has been so estimated that the protein forming the cross linking by the action of AGE causes a dysfunction in various living tissues.

In case of diabetes, it has been reported that a level of nonenzymatic glycosylated protein increases in proportion to an increase of blood sugar and the increase of glycosylated protein becomes one of causes on complications of diabetes [A. Cerami et al, "Metabolism", Vol. 28, pages 431 - 437 (1979); V. M Monier "New England Journal of Medicine", Vol. 314, pages 403 - 408 (1986)]. This course has also been estimated as one of causes of senile deterioration, and for instance, it has been confirmed that the AGE concerns to senile cataract, atherosclerosis, hypertrophy of basement membrane in finer vein due to senile deterioration, and hypertrophy of basement membrane in nephroglomerulus, which causes nephrodysfunction [M. Brownlee et al, "Science", Vol. 232, pages 1629 - 1632 (1986)].

Brownlee et al report in said literature of "Science" a fact that aminoguanidine suppress the Maillard reaction, whereby the compound has been remarked as a drug for preventing age-associated diseases. However, the utility of aminoguanidine is offset by its toxicity, and the toxicity becomes a great disadvantage in clinical use of the compound, since continuous and long period administration shall be required for preventing and curing said diseases including age-associated diseases. In other words, no such a substance has not been found that maillard reaction in living body can be effectively inhibited and is excellent in safety.

An object of the invention is to provide a substance or compound which shows an excellent action to inhibit Maillard reaction and a safety in use, whereby prevention and/or curing of various diseases due to complications of diabetes. arteriosclerosis and senile deterioration.

The inventors have energetically studied and investigated to find that hydantoin compounds shown by following formula (II) and salts thereof show a powerful action of Maillard reaction inhibition and has a low toxicity, so that the invention has been established.

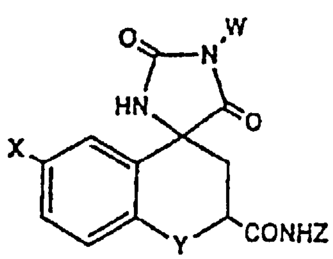

(II)

wherein W is a hydrogen atom or amino radical; X is hydrogen atom, halogen atom, alkyl group having 1 - 6 carbon atoms, or alkoxy group having 1 - 6 carbon atoms; Y is an oxygen atom or sulfur atom; and Z is a hydrogen atom, -NHR group,

in which R is a hydrogen atom, alkyl group having 1 - 6 carbon atoms, alkanoyl group having 1 - 6 carbon atoms, group of

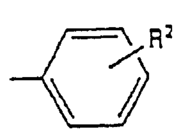

($R^2$ is a hydrogen atom, halogen atom, alkyl group having 1 - 6 carbon atoms or alkoxy group having 1 - 6 carbon atoms),

naphthyl radical, pyridyl radical, furyl radical, thienyl radical, methylphenylsulfonyl radical, $-COCF_3$ radical, $-COOR^1$ group,

($R^1$ is an alkyl having 1 - 6 carbon atoms) or $-COCH(NH_2)R^3$ group,

($R^3$ is a hydrogen atom or an alkyl group having 1 -6 carbon atoms),

but W and Z do not represent same meaning of hydrogen atom.

In the compounds for the use according to the invention, the halogen atom may be of fluorine, chlorine, bromine or iodine. The alkyl group is those having straight-, branched- or cyclic-chain, each of which having 1 - 6 carbon atoms. The alkoxy group is also those having straight-, branched- or cyclic-chain, each of which having 1 - 6 carbon atoms. The alkanoyl group is those having straight- or branched-chain, each of which having 1 - 6 carbon atoms. The salt means those acceptable in the pharmacological field, and as concrete examples, those with a cation such as sodium, potassium, magnesium or the like, with a mineral acid such as hydrogen chloride, sulfuric acid, hydrogen bromide or the like, with an organic acid such as fumaric acid, maleic acid or the like may be listed. Each of the compounds according to the invention includes 4 kinds of stereo-isomers and racemic compound as a mixture of the isomers.

Among the compounds of Formula (II), hydantoin derivatives of

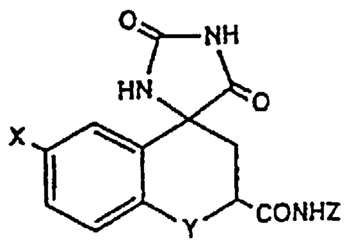

(III)

wherein X is a hydrogen atom, halogen atom, alkyl group having 1 - 6 carbon atoms or alkoxy group having 1 - 6 carbon atoms;, Y is an oxygen atom or sulfur atom; and Z is -NHR group,

in which R is a hydrogen atom, alkyl group having 1 - 6 carbon atoms, group of

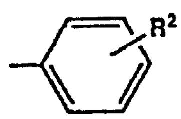

($R^2$ is a hydrogen atom, alkyl group having 1 - 6 carbon atoms, halogen atom or alkoxy group having 1 - 6 carbon atoms),

naphthyl radical, pyridyl radical, furyl radical or thienyl radical,

and salts thereof show an inhibition to the enzyme of aldose reductase, and a patent application thereon has been filed by the assignee company, as useful compounds for preventing and/or curing complications due to diabetes [Jap. Pat. No. Hei 3 (A.D. 1991) - 106885(A), which corresponds to a part of USSN 582039 (USP 5164391) and EP-0 418 834(A1)].

The present inventors have checked an inhibition to the Maillard reaction on these compounds and derivatives thereof to find that all compounds have an activity thereon. Among them, compounds of

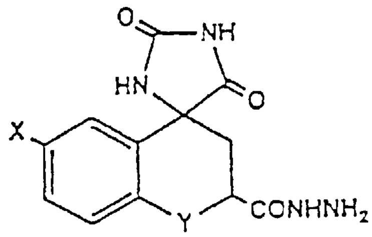

(IV)

wherein X is a hydrogen atom, halogen atom, alkyl group having 1 - 6 carbon atoms or alkoxy group having 1 - 6 carbon atoms; and Y is an oxygen atom or sulfur atom,
show a powerful activity and thus can be expected as a Maillard reaction inhibitor.

Among the compounds of Formula II, there are compounds of the formula

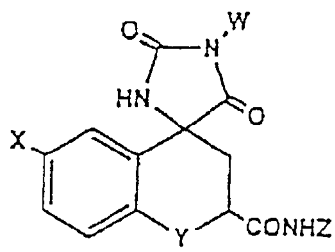

(I)

wherein W is a hydrogen atom or amino radical; X is hydrogen atom, halogen atom, alkyl group having 1 - 6 carbon atoms, or alkoxy group having 1 - 6 carbon atoms; Y is an oxygen atom or sulfur atom; and Z is a hydrogen atom or -NHR group,

in which R is a hydrogen atom, alkanoyl group having 1 - 6 carbon atoms, methylphenylsulfonyl radical, -$COCF_3$ radical, -$COOR^1$ group,

($R^1$ is an alkyl group having 1 - 6 carbon atoms) or -$COCH(NH_2)R^3$ group,

($R^3$ is a hydrogen atom or an alkyl group having 1 - 6 carbon atoms),

but W and Z do not represent same meaning of hydrogen atom, and excluding the compound with 2S,4S configuration, when W represents a hydrogen atom and Z represents -$NH_2$,
and salts thereof.

In this Description and Claims, the designations to be described in italics are given by normal English letters with underlines.

Moreover, it has been found that among the compounds shown by Formula (I), compounds of Formula

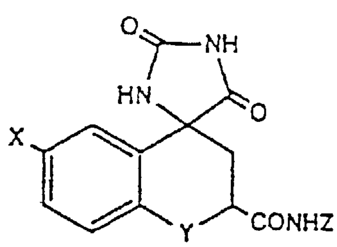

(V)

wherein X is a hydrogen atom, halogen atom, alkyl group having 1 - 6 carbon atoms, or alkoxy group having 1 - 6 carbon atoms; Y is an oxygen atom or sulfur atom; and Z is -NHR group,

in which R is an alkanoyl group having 1 - 6 carbon atoms methylphenylsulfonyl radical, -$COCF_3$ radical, -$COOR^1$ group,

($R^1$ is an alkyl group having 1 - 6 carbon atoms), or -$COCH(NH_2)R^3$ group,

($R^3$ is a hydrogen atom or an alkyl group having 1 - 6 carbon atoms),

and a salt thereof are quite useful as a prodrug of the compounds (IV), and that those provide an improved absorptiveness (increasing a biological availability), elongation of duration of action (repository in drug effect), and the like.

The compounds shown by Formula (I) can easily be prepared by one of following routes.

Route A

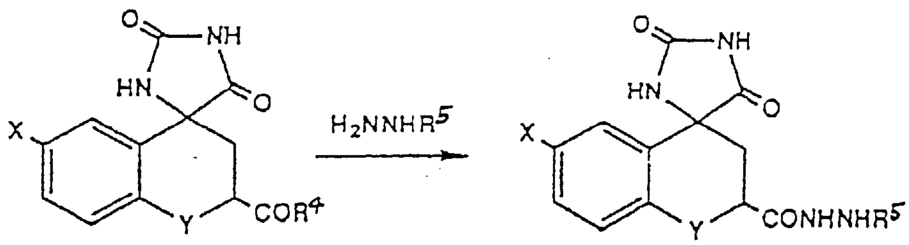


wherein $R^4$ is a halogen atom or alkoxy group having 1 - 6 carbon atoms; $R^5$ is a hydrogen atom, alkanoyl group having 1 - 6 carbon atoms, alkoxycarbonyl group, methylphenylsulfonyl radical, trifluoromethylcarbonyl radical, or aminoalkanoyl group (including protection and deprotection of amino group); and X and Y have the meanings as referred to.

Route B

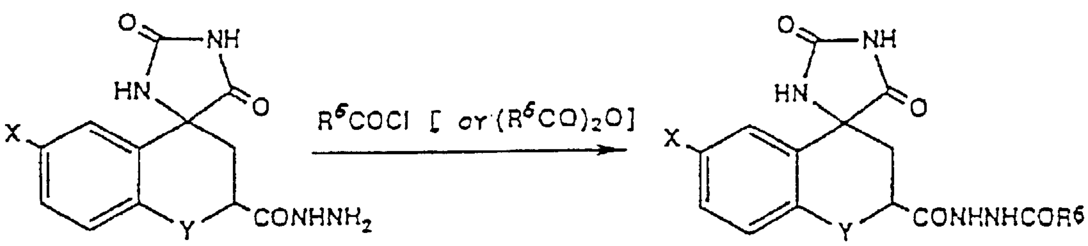


wherein $R^6$ is trifluoromethyl radical or aminoalkyl group (including protected and deprotected amino group); and X and Y have the meanings as referred to.

Route C

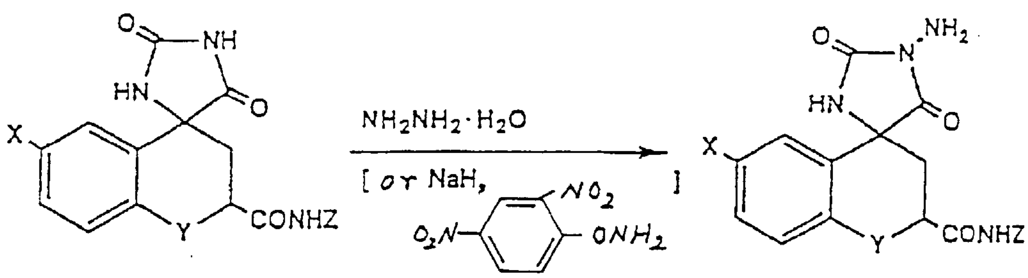

wherein Z is a hydrogen atom or amino radical; and X and Y have the meanings as referred to.

The starting materials for carrying out the process in each Route can easily be synthesized by processes disclosed in Jap. Pat. Nos. Sho 63 (A.D. 1988) - 57588(A) and Hei 3 (A.D. 1991) - 106885(A).

There is no specific limitation, when the derivative or salt according to the invention will be made into a medicine containing at least one of them, as an effective ingredient. Therefore, the medicine may be of a solid form such as a tablet, pill, capsule, powder, granule and suppository, or a liquid form of a solution, suspension or emulsion, together with a conventional additive(s) and/or carrier(s). The medicine of such a form can be prepared in a conventional manner.

For preparing the medicine of solid form, starch, lactose, glucose, calcium phosphate, magnesium stearate, gum arabic or a like vehicle may be used, and if necessary, a lublicant, binder, disintegrating agent, coating agent, coloring agent, flavor and the like may be added. For preparing the medicine of liquid form, a stabilizer, an assistance for dissolving, suspendizer, emulsifier, buffer, reserving agent or the like may be used.

A dosing amount of the derivative or salt thereof for human being depends on kinds of the selected compound or salt, form of the medicine, condition of illness, age of the patient and other factors, but in case of an adult, 0.1 - 500mg/day and more particularly 1 - 150mg/day.

The invention will now be explained in more detail and concretely with reference to Reference Examples, Preparation Examples, Pharmacological Test Examples as well as Prescription Examples.

Reference Example 1

(2S,4S)-6-Fluoro-2',5'-dioxospiro[chroman-4,4'-imidazolidine]-2-carboxylic acid n-propyl ester

To a mixture of (2S,4S)-6-fluoro-2',5'-dioxospiro[chroman-4,4'-imidazolidine]-2-carboxylic acid (5.0g, 18mmol), n-propyl alcohol (20ml, 268mmol) and benzene (5.0ml, 56mmol), was added dropwise concentrated sulfuric acid (0.125ml, 2.35mmol) and then refluxed for 5 hours, while azeotropically removing water by setting a water separator. The reaction mixture was concentrated to half volume and partitioned between 50ml of 5% aqueous solution of sodium bicarbonate and 100ml of ethyl acetate. The organic layer was separated from the aqueous layer, dried over anhydrous sodium sulfate, and evaporated in vacuo to dryness. To the remaining residue, 50ml of water were added and then the aqueous solution was stirred for an hour. The resulting crystals were obtained by a filtration and dried to give 5.60g of the desired compound (Yield : 97.1%).

Melting point : 197 - 200°C .

IR spectrum ($\upsilon_{max}$, KBr) $cm^{-1}$ : 3340, 3265, 1788, 1750, 1720. MS spectrum (EI/DI) m/z : 322 ($M^+$), 192.

$^1$H-NMR spectrum (DMSO-$d_6$) δ ppm :

| | |
|---|---|
| 0.92 | (3H, t), |
| 1.68 | (2H, sextet), |
| 2.21, 2.63 | (2H, m), |
| 4.20 | (2H, t), |
| 5.38 | (1H, dd), |
| 6.90 - 7.50 | (3H, m), |
| 8.48 | (1H, s), |
| 11.10 | (1H, s). |

$[\alpha]_D$, 26°C : +165° (c = 1.0, methanol).

Reference Example 2

(2S,4S)-6 -Fluoro-2',5'-dioxospiro[chroman-4,4'-imidazolidine]-2-carbohydrazide

To a solution of (2S,4S)-6-fluoro-2',5'-dioxospiro[chroman-4,4'-imidazolidine]-2-carboxylic acid n-propyl ester (9.25g, 28.7mmol) obtained by the process described in Reference Example 1 in n-propyl alcohol (24.5ml, 327mmol) and toluene (18ml, 169mmol), was added hydrazine hydrate (2.21ml, 57.4mmol) and then refluxed for 7 hours. The reaction mixture was stirred at 15 - 20°C for 15 hours. The resulting crystals were obtained by filtration and washed with 30ml of n-propyl alcohol and recrystallized from ethanol to give 5.77g of the desired compound (Yield : 68.4%).

Melting point : 277 - 278°C.

IR spectrum ($\upsilon_{max}$, KBr) $cm^{-1}$ :
3450, 3330, 3060, 1775, 1725, 1660.

MS spectrum (EI/DI) m/z :
294 ($M^+$), 235, 192.
$^1$H-NMR spectrum (DMSO-$d_6$) δ ppm :

| | |
|---|---|
| 2.13 | (1H, dd), |
| 2.40 | (1H, dd), |
| 4.39 | (2H, s), |
| 5.10 | (1H, dd), |
| 6.9 - 7.2 | (3H, m), |
| 8.38 | (1H, s), |
| 9.59 | (1H, s), |
| 11.00 | (1H, s). |

$[\alpha]_D$, 25°C : +139° (c = 1.0, methanol).

Reference Example 3

(2S,4S)-6-Fluoro-N'-methyl-2',5'-dioxospiro[chroman-4,4'-imidazolidine]-2-carbohydrazide

To a solution of (2S,4S)-6-fluoro-2',5'-dioxospiro[chroman-4,4'-imidazolidine]-2-carboxylic acid n-propyl ester (30.0g, 93.lmmol) obtained by the process described in Reference Example 1 in n-propyl alcohol (800ml), was added methylhydrazine (44.2g, 931mmol). After refluxing the mixture for 18 hours under an argon atmosphere, the reaction mixture was concentrated to half volume and formed precipitate was removed by filtration. The filtrate was evaporated in vacuo to dryness and to the residue, was added 100ml of water. The resulting crystals were obtained by filtration and dried to give 20.3g of the desired compound (Yield : 70.9%).

Melting point : 276 - 278°C.
IR spectrum ($\upsilon_{max}$, KBr) $cm^{-1}$ : 3430, 3060, 1775, 1730, 1660.
MS spectrum (EI/DI) m/z : 308 ($M^+$), 278, 235, 192.
$^1$H-NMR spectrum (DMSO-$d_6$) δ ppm :

| | |
|---|---|
| 2.12 | (2H, dd), |
| 2.41 | (1H, dd), |
| 2.47 | (3H, s), |
| 4.97 | (1H, brs), |
| 5.09 | (1H, dd), |
| 6.9 - 7.2 | (3H, m), |
| 8.39 | (1H, s), |
| 9.85 | (1H, s), |
| 11.02 | (1H, s). |

$[\alpha]_D$, 25°C +137° (c = 1.0, methanol).

Reference Example 4

(2S,4S)-6-Fluoro-2',5'-dioxo-N'-phenylspiro[chroman-4,4'-imidazolidine]-2-carbohydrazide

To thionyl chloride (24.9ml, 3.57mol), was added (2S,4S)-6-fluoro-2',5'-dioxospiro[chroman-4,4'-imidazolidine]-2-carboxylic acid (20.0g, 71.4mmol). After refluxing the mixture under an argon atmosphere for 22 hours, the excess thionyl chloride was evaporated in vacuo to dryness to give quantitatively crude crystals of (2S,4S)-6-fluoro-2',5'-dioxospiro[chroman-4,4'-imidazolidine]-2-carbonyl chloride. To a solution of this acid chloride in 200ml of N,N-dimethylformamide (DMF), were added phenylhydrazine (15.6g, 143mmol) and triethylamine (14.4g, 143mmol). After stirring the mixture for 18 hours at 25°C, 600ml of water were added to the reaction mixture. The resulting mixture was extracted with ethyl acetate. The organic layer was washed with water, dried over anhydrous sodium sulfate, filtered and evaporated in vacuo to dryness. The resulting residue was chromatographed on silica gel, eluted with $CH_2Cl_2$ : MeOH = 20 : 1 to give colorless crystals. The crystals were recrystallized from 50% aqueous ethanol to give colorless

needles of the desired compound (Yield : 17.4g, 65.9%).

Melting point : 254 - 255°C.

IR spectrum ($\upsilon_{max}$, KBr) $cm^{-1}$ :
3520, 3400, 3060, 1775, 1735, 1670.

MS spectrum (EI/DI) m/z :
370 ($M^+$), 307, 278, 235, 192.

$^1H$-NMR spectrum (DMSO-$d_6$) δ ppm :

| | |
|---|---|
| 2.19 | (1H, dd), |
| 2.52 | (1H, dd), |
| 5.20 | (1H, dd), |
| 6.7 - 6.8 | (5H, m), |
| 6.9 - 7.2 | (3H, m), |
| 7.84 | (1H, s), |
| 8.39 | (1H, s), |
| 10.25 | (1H, s), |
| 11.02 | (1H, s). |

$[\alpha]_D$, 25°C +130° (c = 1.0, methanol).

Reference Example 5

(2S, 4S)-N'-(4-Chlorophenyl)-6-fluoro-2',5'-dioxospiro[chroman-4,4'-imidazolidine]-2-carbohydrazide

This compound was prepared by the same procedure as in the case of Reference Example 4, except that 4-chlorophenylhydrazine (20.4g, 143mmol) was employed for phenylhydrazine to obtain the desired compound (Yield 21.7g, 75.1%).

Melting point : 169 - 171°C.

IR spectrum ($\upsilon_{max}$, KBr) $cm^{-1}$ : 3510, 3400, 3060, 1775, 1735, 1670.

MS spectrum (EI/DI) m/z : 404 ($M^+$), 192.

$^1H$-NMR spectrum (DMSO-$d_6$) δ ppm :

| | |
|---|---|
| 2.19 | (1H, dd), |
| 2.50 | (1H, dd), |
| 5.26 | (1H, dd), |
| 6.9 - 7.2 | (3H, m), |
| 7.18 | (2H, d), |
| 8.00 | (1H, s), |
| 8.36 | (1H, s), |
| 10.27 | (1H, s), |
| 11.01 | (1H, s). |

$[\alpha]_D$, 25°C : +121° (c = 1.0, methanol).

Reference Example 6

(2S,4S)-6-Fluoro-N'-(4-methoxyphenyl)-2',5'-dioxospiro[chroman-4,4'-imidazolidine]-2-carbohydrazide

This compound was prepared by the same procedure as in the case of Reference Example 4, except that 4-methoxyphenylhydrazine (19.7g, 143mmol) was employed for phenylhydrazine to obtain the desired compound (Yield : 22.3g, 78.0%).

Melting point : 154 - 157°C .

IR spectrum ($\upsilon_{max}$, KBr) $cm^{-1}$ :
3400, 3060, 1775, 1730, 1690.

MS spectrum (EI/DI) m/z :

400 ($M^+$), 137.

$^1$H-NMR spectrum (DMSO-$d_6$) δ ppm :

| | |
|---|---|
| 2.22 | (1H, dd), |
| 2.50 | (1H, dd), |
| 5.23 | (1H, dd), |
| 6.6 - 7.2 | (7H, m), |
| 7.51 | (1H, d), |
| 8.38 | (1H, s), |
| 10.22 | (1H, d), |
| 11.03 | (1H, s). |

$[\alpha]_D$, 25°C +127° (c = 1.0, methanol).

Reference Example 7

(2S,4S)-6-Fluoro- N'-(1-naphthyl)-2',5'-dioxospiro[chroman-4,4'-imidazolidine]-2-carbohydrazide

This compound was prepared by the same procedure as in the case of Reference Example 4, except that 1-naphthylhydrazine (20.4g, 143mmol) was employed for phenylhydrazine to obtain the desired compound (Yield : 22.7g, 75.8%).

Melting point : 285 - 288°C

IR spectrum ($\upsilon_{max}$, KBr) $cm^{-1}$ : 3370, 3330, 3060, 1780, 1735, 1680.

MS spectrum (EI/DI) m/z : 420 ($M^+$), 143.

$^1$H-NMR spectrum (DMSO-$d_6$) δ ppm :

| | |
|---|---|
| 2.27 | (1H, dd), |
| 2.55 | (1H, dd), |
| 5.36 | (1H, dd), |
| 6.7 - 8.2 | (10H, m), |
| 8.34 | (1H, s), |
| 8.38 | (1H, s), |
| 10.40 | (1H, s), |
| 11.03 | (1H, s). |

$[\alpha]_D$, 25°C : +131° (c = 1.0, methanol).

Example 1

(2R,4R)-6-Fluoro-2',5'-dioxospiro[chroman-4,4'-imidazolidine]-2-carbohydrazide

This compound was prepared by the same procedure as in the case of Reference Example 2, except that (2R,4R)-6-fluoro-2',5'-dioxospiro[chroman-4,4'-imidazolidine]-2-carboxylic acid n-propyl ester (9.25g, 28.7mmol) was employed as the starting material. In this case, the desired compound (Yield : 5.33g, 63.2%) was obtained.

Melting point : 277 - 278°C .

IR spectrum ($\upsilon_{max}$, KBr) $cm^{-1}$ : 3450, 3330, 3060, 1775, 1725, 1660.

MS spectrum (EI/DI) m/z : 294 ($M^+$).

$^1$H-NMR spectrum (DMSO-$d_6$) δ ppm :

| | |
|---|---|
| 2.14, 2.39 | (2H, m), |
| 4.36 | (2H, s), |
| 5.11 | (1H, dd), |
| 6.93 - 7.17 | (3H, m), |
| 8.33 | (1H, s), |
| 9.54 | (1H, s), |

(continued)

| | |
|---|---|
| 10.98 | (1H, s). |

$[\alpha]_D$, 25°C : -161° (c = 1.0, methanol).

Example 2

(2R,4S)-6-Fluoro-2',5'-dioxospiro[chroman-4,4'-imidazolidine]-2-carbohydrazide

This compound was prepared by the same procedure as in the case of Reference Example 2, except that (2R,4S)-6-fluoro-2',5'-dioxospiro[chroman-4,4'-imidazolidine]-2-carboxylic acid n-propyl ester (9.25g, 28.7mmol) was employed as the starting material. In this case, the desired compound (Yield : 4.00g, 47.4%) was obtained.

Melting point : 171 - 175°C .

IR spectrum ($\upsilon_{max}$, KBr) $cm^{-1}$ : 3430, 3330, 3060, 1775, 1725, 1665.

MS spectrum (EI/DI) m/z :
294 ($M^+$).

$^1$H-NMR spectrum (DMSO-$d_6$) δ ppm :

| | |
|---|---|
| 2.22, 2.34 | (2H, m), |
| 4.37 | (2H, s), |
| 4.57 | (1H, dd), |
| 6.84 - 7.21 | (3H, m), |
| 8.84 | (1H, s), |
| 9.48 | (1H, brs), |
| 10.98 | (1H, br). |

$[\alpha]_D$, 25°C : -99° (c = 1.0, methanol).

Example 3

(2S,4R)-6-Fluoro-2',5'-dioxospiro[chroman-4,4'-imidazolidine]-2-carbohydrazide

This compound was prepared by the same procedure as in the case of Reference Example 2, except that (2S,4R)-6-fluoro-2',5'-dioxospiro[chroman-4,4'-imidazolidine]-2-carboxylic acid n-propyl ester (9.25g, 28.7mmol) was employed as the starting material. In this case, the desired compound (Yield : 3.64g, 43.1%) was obtained.

Melting point : 168 - 171°C.

IR spectrum ($\upsilon_{max}$, KBr) $cm^{-1}$ : 3430, 3330, 3050, 1775, 1725, 1665.

MS spectrum (EI/DI) m/z : 294 ($M^+$).

$^1$H-NMR spectrum (DMSO-$d_6$) δ ppm :

| | |
|---|---|
| 2.22, 2.34 | (2H, m), |
| 4.37 | (2H, s), |
| 4.57 | (1H, dd), |
| 6.83 - 7.21 | (3H, m), |
| 8.83 | (1H, s), |
| 9.48 | (1H, brs), |
| 10.98 | (1H, br). |

$[\alpha]_D$, 25°C : +97° (c = 1.0, methanol).

Example 4

(2S,4S)-6-Fluoro- N'-(4-methylphenylsulfonyl)-2',5'-dioxospiro[chroman-4,4'-imidazolidine]-2-carbohydrazide

To thionyl chloride (70ml, 960mmol), was added (2S,4S)-6-fluoro-2',5'-dioxospiro[chroman-4,4'-imidazolidine]-2-carboxylic acid (6.85g, 24.5mmol). After refluxing the mixture for 18 hours, the excess thionyl chloride was evaporated in vacuo to dryness to give a residue of (2S,4S)-6-fluoro-2',5'-dioxospiro[chroman-4,4'-imidazolidine]-2-carbonyl chloride. To a solution of this acid chloride in 70ml of DMF, were added triethylamine (3.90ml, 28.0mmol) and p-toluenesulfonyl hydrazide (4.56g, 24.5mmol). After stirring the mixture for 4 hours at 20 - 25°C, 30ml of water was added to the reaction mixture. The solution was then acidified to pH 1 with IN-hydrochloric acid and extracted with ethyl acetate. The organic layer was dried over anhydrous sodium sulfate, filtered and evaporated in vacuo to dryness. The resulting residue (6.66g) was chromatographed on silica gel, eluted with AcOEt : n-hexane = 2 : 1 to give colorless crystals of the desired compound (Yield : 5.09g, 46.4%).

Melting point : 164 - 166°C .

IR spectrum ($\upsilon_{max}$, KBr) $cm^{-1}$ :
3264, 1779, 1732, 1597, 1491, 1165.

MS spectrum (EI/DI) m/z :
448 ($M^+$), 390, 264, 193.

$^1$H-NMR spectrum (DMSO-$d_6$) δ ppm :

| | |
|---|---|
| 1.95 | (1H, dd), |
| 2.23 | (1H, dd), |
| 2.38 | (3H, s), |
| 5.07 | (1H, dd), |
| 6.97 | (2H, m), |
| 7.15 | (1H, td), |
| 7.37 | (2H, d), |
| 7.71 | (2H, d), |
| 8.37 | (1H, s), |
| 9.98 | (1H, s), |
| 10.65 | (1H, s), |
| 11.05 | (1H, s). |

Example 5

(2S, 4S)-N'-tert-Butoxycarbonyl-6-fluoro-2',5'-dioxospiro[chroman-4,4'-imidazolidine]-2-carbohydrazide

To thionyl chloride (100ml, 1.37mol), was added (2S,4S)-6-fluoro-2',5'-dioxospiro[chroman-4,4'-imidazolidine]-2-carboxylic acid (10.0g, 35.7mmol). After refluxing the mixture for 18 hours, the excess thionyl chloride was evaporated in vacuo to dryness to give a residue of (2S,4S)-6-fluoro-2',5'-dioxospiro[chroman-4,4'-imidazolidine]-2-carbonyl chloride. To a solution of this acid chloride in 100ml of DMF, were added triethylamine (9.94ml, 71.4mmol) and tert-butoxycarbazate (4.71g, 35.6mmol). After stirring the mixture for 4 hours at 20 - 25°C . 100ml of water was added to the reaction mixture. The solution was then acidified to pH 3 with 1N-hydrochloric acid and extracted with ethyl acetate. The organic layer was dried over anhydrous sodium sulfate, filtered and evaporated in vacuo to dryness. The resulting residue (6.40g) was chromatographed on silica gel, eluted with AcOEt : n-hexane = 2 : 1 to give colorless crystals of the desired compound (Yield : 4.80g, 34.1%).

Melting point : 165 - 167°C .

IR spectrum ($\upsilon_{max}$, KBr) $cm^{-1}$ : 3281, 1779, 1732, 1491, 1262, 1163.

MS spectrum (EI/DI) m/z : 294 ($M^+$-100), 235, 192, 164.

$^1$H-NMR spectrum (DMSO-$d_6$) δ ppm :

| | |
|---|---|
| 1.41 | (9H, s), |
| 2.11 | (1H, t), |
| 2.44 | (1H, d), |
| 5.16 | (1H, d), |
| 7.00 | (2H, m), |
| 7.17 | (1H, td), |
| 8.42 | (1H, s), |
| 8.93 | (1H, s), |
| 10.13 | (1H, s), |

(continued)

| | |
|---|---|
| 11.08 | (1H, s). |

Example 6

(2S,4S)-N'-Acetyl-6-fluoro-2',5'-dioxospiro[chroman-4,4'-imidazolidine]-2-carbohydrazide

To thionyl chloride (100ml, 1.37mol), was added (2S,4S)-6-fluoro-2',5'-dioxospiro[chroman-4,4'-imidazolidine]-2-carboxylic acid (10.0g, 35.7mmol). After refluxing the mixture for 17 hours, the excess thionyl chloride was evaporated in vacuo to give a residue of (2S,4S)-6-fluoro-2',5'-dioxospiro[chroman-4,4'-imidazolidine]-2-carbonyl chloride. A solution of this acid chloride in 50.0ml of DMF was added dropwise into a solution of triethylamine (6.00ml, 43.lmmol) and acetohydrazide (2.65g, 35.8mmol) in 50.0ml of DMF. After stirring the mixture for 19 hours at 20 - 25°C, the reaction mixture was evaporated in vacuo to give a residue. To the resulting residue, was added 100ml of water and the solution was stirred. The resulting crystals was filtered and recrystallized from methanol to give colorless crystals of the desired compound (Yield : 7.71g, 64.3%).

Melting point : 286 - 295°C.

IR spectrum ($\upsilon_{max,}$ KBr) $cm^{-1}$ : 3403, 3355, 3182, 1781, 1734, 1637, 1489.

MS spectrum (EI/DI) m/z : 336 ($M^+$), 294, 262, 192.

$^1H$-NMR spectrum (DMSO-$d_6$) δ ppm :

| | |
|---|---|
| 1.88 | (3H, s), |
| 2.15 | (1H, dd), |
| 2.46 | (1H, dd), |
| 5.20 | (1H, dd), |
| 7.00 | (2H, m), |
| 7.17 | (1H, td), |
| 8.44 | (1H, s), |
| 9.90 | (1H, s), |
| 10.25 | (1H, s), |
| 11.18 | (1H, s). |

Example 7

(2S, 4S)-6-Fluoro-2',5'-dioxo-N'-trifluoroacetylspiro[chroman-4,4'-imidazolidine]-2-carbohydrazide

(2S,4S)-6-Fluoro-2',5'-dioxospiro[chroman-4,4'-imidazolidine]-2-carbohydrazide (8.00g, 27.2mmol) was dissolved in 80ml of absolute pyridine under an argon atmosphere and cooled to -20°C . To the solution, trifluoroacetic anhydride (3.90ml, 27.6mmol) was added dropwise and slowly. After stirring the solution at 5 - 15°C for 20 hours, the solvent was evaporated in vacuo, and oily residue was suspended in a solution of water-ethyl acetate. The resulting crystals were filtered and dried to give colorless crystals of the desired compound (Yield : 10.6g, 99.3%).

Melting point : 167 - 169°C.

IR spectrum ($\upsilon_{max,}$ KBr) $cm^{-1}$ : 3426, 3283, 3227, 1726, 1692, 1570, 1491, 1213, 1194, 1165.

MS spectrum (EI/DI) m/z :

390 ($M^+$), 262, 234, 219, 192.

$^1H$-NMR spectrum (DMSO-$d_6$) δ ppm :

| | |
|---|---|
| 2.13 | (1H, t), |
| 2.53 | (1H, d), |
| 5.27 | (1H, d), |
| 7.03 | (2H, m), |
| 7.19 | (1H, td), |
| 8.43 | (1H, s), |
| 10.80 | (1H, s), |
| 11.10 | (1H, s), |

(continued)

| | |
|---|---|
| 11.52 | (1H, s). |

Reference Example 8

N-tert-Butoxycarbonyl-L-alanyl hydrazide

N-tert-Butoxycarbonyl-L-alanine (10.0g, 52.9mmol) and potassium carbonate (10.2g, 73.8mmol) were suspended in 100ml of acetone, and dimethyl sulfate (6.00ml, 63.4mmol) was added dropwise into the suspension. After stirring at 10 - 15°C for 15 hours, 5.0ml of methanol was added to the reaction mixture. The resulting crystals were filtered off and filtrate was evaporated in vacuo. To the residue, was added dichloromethane, and undissolved matter was filtered off. The filtrate was evaporated in vacuo to give colorless oily products of methyl N-tert-butoxycarbonyl-2-aminopropionate (10.7g, quant.). The oily products was dissolved in 50ml of methanol, and hydrazine monohydrate (10.3ml, 212mmol) was added to the solution. After stirring at 15 - 20°C for 21 hours, the solvent was evaporated in vacuo and the remaining residue was recrystallized from ethyl acetate-n-hexane to give colorless needles of the desired compound (Yield : 10.7g, quant.).

IR spectrum ($\upsilon_{max,}$ KBr) $cm^{-1}$ : 3331, 1691, 1663, 1617, 1592.

MS spectrum (EI/DI) m/z : 204 [$(M + H)^+$], 148, 104.

$^1$H-NMR spectrum (DMSO-$d_6$) δ ppm :

| | |
|---|---|
| 1.13 | (3H, d), |
| 1.36 | (9H, s), |
| 3.92 | (1H, m), |
| 4.18 | (2H, s), |
| 6.85 | (1H, d), |
| 8.98 | (1H, s). |

Reference Example 9

(2S,4S)- N'-(N-tert-Butoxycarbonyl-L-alanyl)-6-fluoro-2',5'-dioxospiro[chroman-4,4'-imidazolidine]-2-carbohydrazide

To thionyl chloride (100ml, 1.37mol), was added (2S,4S)-6-fluoro-2',5'-dioxospiro[chroman-4,4'-imidazolidine]-2-carboxylic acid (10.0g, 35.7mmol). After refluxing the mixture for 16 hours, the excess thionyl chloride was evaporated in vacuo to dryness to give a residue of (2S,4S)-6-fluoro-2',5'-dioxospiro[chroman-4,4'-imidazolidine]-2-carbonyl chloride. A solution of this acid chloride in 50.0ml of DMF was added dropwise into a solution of triethylamine (6.00ml, 43.lmmol) and N-tert-butoxycarbonyl-L-alanyl hydrazide (7.98g, 39.3mmol) in 50.0ml of DMF. After stirring the mixture for 17 hours at 20 - 25°C, the reaction mixture was evaporated in vacuo to dryness. To the remaining residue, were added 200ml of water, and the aqueous solution was extracted with ethyl acetate. The organic layer was dried over anhydrous sodium sulfate, filtered and evaporated in vacuo to dryness. The resulting residue was chromatographed on silica gel, eluted with $CH_2Cl_2$ : MeOH = 20 : 1 to give colorless crystals which were recrystallized from ethyl acetate to give colorless crystals of the desired compound (Yield : 5.75g, 34.6%).

IR spectrum ($\upsilon_{max,}$ KBr) $cm^{-1}$ : 3279, 1776, 1730, 1491, 1262, 1167.

MS spectrum (EI/DI) m/z : 347 ($M^+$-118), 330.

$^1$H-NMR spectrum (DMSO-$d_6$) δ ppm :

| | |
|---|---|
| 1.23 | (3H, d), |
| 1.38 | (9H, s), |
| 2.17 | (1H, dd), |
| 2.44 | (1H, dd), |
| 4.06 | (1H, m), |
| 5.21 | (1H, dd), |
| 6.92 | (1H, m), |
| 6.99 | (2H, m), |
| 7.15 | (1H, td), |

(continued)

| | |
|---|---|
| 8.36 | (1H, s), |
| 9.87 | (1H, s), |
| 10.28 | (1H, s), |
| 10.99 | (1H, s). |

Example 8

(2S, 4S)- N'-L-Alanyl-6-fluoro-2',5'-dioxospiro[chroman-4,4'-imidazolidine]-2-carbohydrazide

(2S ,4S)-N'-(N-tert-Butoxycarbonyl-L-alanyl)-6-fluoro-2',5-dioxospiro[chroman-4,4'-imidazolidine]-2-carbohydrazide (5.37 g, 11.5 mmol) was added to trifluoroacetic acid (25.0ml, 0.324mol) at -20°C and this solution was stirred for 30 minutes at room temperature. Trifluoroacetic acid was evaporated in vacuo to dryness. The resulting residue was dissolved in 1,4-dioxane and chromatographed on Amberlyst A-21, eluted with 1,4-dioxane to give colorless crystals of the desired compound (Yield : 4.21g, quant.).

Melting point : 191 - 195°C .

IR spectrum ($\upsilon_{max,}$ KBr) $cm^{-1}$ : 3225, 3058, 1772, 1726, 1678, 1491, 1206.

MS spectrum (EI/DI) m/z : 365 ($M^+$), 347, 330, 294, 262, 193.

$^1$H-NMR spectrum (DMSO-$d_6$) $\delta$ ppm :

| | |
|---|---|
| 1.29 | (3H, d), |
| 2.16 | (1H, dd), |
| 2.47 | (1H, dd), |
| 3.66 | (1H, q), |
| 5.24 | (1H, dd), |
| 7.00 | (2H, m), |
| 7.18 | (1H, td), |
| 8.44 | (1H, s). |

Example 9

(2S,4S)-1'-Amino-6-fluoro-2',5'-dioxospiro[chroman-4,4'-imidazolidine]-2-carbohydrazide

(2S,4S)-6-Fluoro-2',5'-dioxospiro[chroman-4,4'-imidazolidine]-2-carbohydrazide (1.00g, 3.40mmol) and hydrazine monohydrate (1.70g, 34.0mmol) were dissolved into 20.0ml of n-propanol by heating the same, and the resulting solution was refluxed for 60 hours. The reaction mixture was evaporated in vacuo, and the remaining residue was chromatographed on silica gel, eluted with $CH_2Cl_2$ : MeOH = 10 1 to give colorless crystals of the desired compound (Yield : 768mg, 73.0%).

IR spectrum ($\nu_{max,}$ KBr) $cm^{-1}$ : 3326, 1781, 1725.

MS spectrum (EI/DI) m/z :

309 ($M^+$), 207.

$^1$H-NMR spectrum (DMSO-$d_6$) $\delta$ ppm :

| | |
|---|---|
| 2.15 | (1H, dd), |
| 2.35 | (1H, dd), |
| 4.36 | (2H, s), |
| 4.72, 4.74 | (2H, each s), |
| 5.13 | (1H, dd), |
| 6.91 | (1H, dd), |
| 6.99 | (1H, dd), |
| 7.15 | (1H, dt), |
| 8.53 | (1H, s), |
| 9.54 | (1H, s). |

Example 10

(2S,4S)-1'-Ainino-6-fluoro-2',5'-dioxospiro[chroman-4,4'-imidazolidine]-2-carboxamide

(2S,4S)-6-Fluoro-2',5'-dioxospiro[chroman-4,4'-imidazolidine]-2-carboxamide (670mg, 2.40mmol) was dissolved in absolute DMF. Sodium hydride (57. 6mg, 2.40mmol) was added to the solution under an argon atmosphere at 5 - 10°C , and the reaction mixture was stirred at 20°C for 30 minutes. To the resulting solution, was added o-(2,4-dinitrophenyl)hydroxylamine (478mg, 2.40mmol) at 5 - 10°C . The reaction mixture was stirred for an hour at the same temperature, and further for an hour at 20°C, and evaporated in vacuo to dryness at temperature below 50°C. To the remaining residue, 0.1N aqueous hydrochloric acid was added, and the solution was washed with ethyl ether. This acidic solution was alkalized by addition of sodium bicarbonate at 5 - 10 °C, and evaporated in vacuo to dryness. The resulting residue was chromatographed on silica gel, eluted with $CH_2Cl_2$ : MeOH = 50 : 1 - 20 : 1 to give crude crystals which were recrystallized from ethanol to give colorless crystals of the desired compound (Yield : 420mg, 60.0%).

IR spectrum ($\upsilon_{max}$, KBr) $cm^{-1}$ : 3420, 3299, 1786, 1725, 1671.
MS spectrum (EI/DI) m/z : 294 ($M^+$), 207.
$^1$H-NMR spectrum (DMSO-$d_6$) $\delta$ ppm :

| | |
|---|---|
| 2.06 | (1H, t), |
| 2.41 | (1H, dd), |
| 4.73, 4.74 | (2H, each s), |
| 5.05 | (1H, dd), |
| 6.91 | (1H, dd), |
| 7.02 | (1H, dd), |
| 7.15 | (1H, dt), |
| 7.45, 7.69 | (2H, each s), |
| 8.53 | (1H, s). |

Pharmacological Test Example 1

A testing or control compound (5mM), bovine serum albumin (BSA, 10mg/ml) and fructose (400mM) were dissolved in 0.5mM Na-phosphate buffer (pH 7.4) containing 20% DMSO. The mixture was incubated at 37°C for 24 hours.

The incubated mixture was diluted to 10 folds in volume with distilled water and measured fluorescense intensity at excitation wavelength of 350nm and emission wavelength of 428nm with a HITACHI F-4010 spectrofluorometer. The inhibition ratio of fluorescense development was estimated with the following equation.

$$\text{Inhibition } (\%) = \{[F1-F3-F4)- (F2-F3-F4)]/(F1-F3-F4)\} \times 100$$

wherein,

| | |
|---|---|
| Inhibition : | Inhibition ratio, |
| F1 : | Fluorescense intensity of the solution containing no compound, |
| F2 : | Fluorescense intensity of the solution containing the compound, |
| F3 : | Fluorescense intensity of the solution containing only BSA, and |
| F4 : | Fluorescense intensity of the solution containing only fructose. |

Results are shown in following Table 1.

Table 1

| Compound | Inhibition ratio (%) |
|---|---|
| Testing compound | |
| Reference Example | |
| 2 | 88.7 |

Table 1 (continued)

| Compound | Inhibition ratio (%) |
|---|---|
| Testing compound | |
| Reference Example | |
| 3 | 41.6 |
| 4 | 17.8 |
| Example | |
| 1 | 87.3 |
| 2 | 88.6 |
| 3 | 88.1 |
| 7 | 21.3 |
| 9 | 86.6 |
| 10 | 19.7 |
| Control compound | |
| Aminoguanidine | 86.9 |

Pharmacological Test Example 2

A testing or control compound (5mM), fructose (400mM) and a protein of either BSA (10mg/ml) or lysozyme (10mg/ml) were dissolved in 0.5M Na-phosphate buffer (pH 7.4). The mixture was incubated at 37°C for 7 days At the end of the reaction period, sodium dodecyl sulfonate-polyacrylamide gel electrophoresis (SDS-PAGE) was carried out with using aliquots of the reaction mixture. Protein band was stained with Coomassie brilliant blue and protein content of each band was determined with a SHIMAZU UV-265 scanmeter.

The degree of cross-link formation was showed as the ratio between cross-linked protein content and total protein content. The inhibition ratio of cross-linking of proteins was estimated with the following equation.

$$\text{Inhibition ratio (\%)} = [(A - B)/A] \times 100$$

wherein,

A : degree of cross-link formation (containing no compound), and
B : degree of cross-link formation (containing the compound).

Table 2

| Compound | Protein | Inhibition ratio (%) |
|---|---|---|
| Reference Example 2 | BSA | 88.7 |
| | Lysozyme | 67.4 |
| Control Aminoguanidine | BSA | 70.7 |
| | Lysozyme | 59.3 |

Pharmacological Test Example 3 (Study on toxicity)

Acute and 4 week subacute toxicities of exemplar compounds according to the invention were checked by oral administration thereof and using rats.

Results are shown in following Tables 3 and 4. As apparent therefrom, the compounds according to the invention are excellent in safety of use.

Table 3

| Compound | Animal | $LD_{50}$ (mg/kg) |
|---|---|---|
| Reference Example 2 | rat (male) | > 4000 |
| | rat (female) | > 4000 |
| Example 1 | rat (male) | > 4000 |
| | rat (female) | > 4000 |
| Aminoguanidine | rat (male) | 1900 |
| | rat (female) | 1300 |

Table 4

| Compound | Animal | Non-toxic dose |
|---|---|---|
| Reference Example 2 | rat (male) | > 2000 mg/kg |
| Aminoguanidine | rat (male) | 30 mg/kg |

Consideration on Pharmacological Tests

The compounds according to the invention shows an inhibition to formation of Maillard reaction products (fluorescent substance), as shown in Table 1, and an inhibition to polymerization of proteins due to advance of Maillard reaction, as shown in Table 2. Further, the toxicity of compounds is quite low in comparison with that of the control compound of aminoguanidine (see Tables 3 and 4).

Therefore, the compounds according to the invention is useful as an effective ingredient for the medicine to prevent and/or cure an arteriosclerosis, complications due to diabetes and age-concerned diseases, which requires continuous administration for a long period of time.

Prescription Example 1 (Tablets)

1000 Tablets were prepared in a conventional manner with use of following components. Each tablet contains the active ingredient by 50mg.

An amount of the active ingredient in each tablet can be set to 1.0, 4.0, 5.0, 10, 25, 100mg and so on, by changing an amount of the same to be composed.

| | |
|---|---|
| Active ingredient (Example 1) | 50 (g) |
| Sodium citrate | 25 |
| Arginine | 10 |
| Polyvinylpyrrolidone | 10 |
| Magnesium stearate | 5 |

Prescription Example 2 (Capsules)

1000 Capsules were prepared by composing the following ingredients and filling the same into gelatin capsules, in a conventional manner. Each capsule contains the active ingredient by 10mg.

An amount of the active ingredient in each capsule can be set to 1.0, 4.0, 5.0, 25, 50, 100mg and so on, by changing an amount of the same to be composed.

| | |
|---|---|
| Active ingredient (Reference Example 2) | 10 (g) |
| Lactose | 70 |
| Corn starch | 20 |

## Claims

**1.** A hydantoin derivative selected from the group consisting of

(A) (2R,4S)-6-Fluoro-2',5'-dioxospiro[chroman-4,4'-imidazolidine]-2-carbohydrazide,
(B) (2S,4R)-6-Fluoro-2',5'-dioxospiro[chroman-4,4'-imidazolidine]-2-carbohydrazide,
(C) (2R,4R)-6-Fluoro-2',5'-dioxospiro[chroman-4,4'-imidazolidine]-2-carbohydrazide,
(D) (2S, 4S)-N'-Acetyl-6-fluoro-2',5-dioxospiro[chroman-4,4'-imidazolidine]-2-carbohydrazide
(E) (2S, 4S)-6-Fluoro-N'-(4-methylphenylsulfonyl)-2',5'-dioxospiro[chroman-4,4'-imidazolidine]-2-carbohydrazide
(F) (2S, 4S)-N'-tert-Butoxycarbonyl-6-fluoro-2,5-'dioxospiro[chroman-4,4'-imidazolidine]-2-carbohydrazide
(G) (2S, 4S)-N'-L-Alanyl-6-fluoro-2',5'-dioxospiro[chroman-4,4'-imidazolidine]-2-carbohydrazide
(H) (2S, 4S)-6-Fluoro-2',5-dioxo-N'-trifluoroacetylspiro[chroman-4,4'-imidazolidine]-2-carbohydrazide
(I) (2S,4S)-1'-Amino-6-fluoro-2',5'-dioxospiro[chroman-4,4'-imidazolidine]-2-carbohydrazide, and
(J) (2S,4S)-1'-Amino-6-fluoro-2',5'-dioxospiro[chroman-4,4'-imidazolidine]-2-carboxamide,

or a salt of the derivatives.

**2.** The use of a hydantoin derivative of the formula

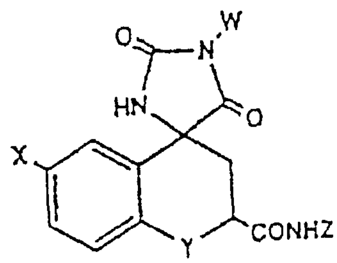

wherein W is a hydrogen atom or amino radical; X is hydrogen atom, halogen atom, alkyl group having 1-6 carbon atoms, or alkoxy group having 1-6 carbon atoms; Y is an oxygen atom or sulfur atom; and Z is a hydrogen atom, -NHR group in which R is a hydrogen atom, alkyl group having 1-6 carbon atoms, alkanoyl group having 1-6 carbon atoms, group of

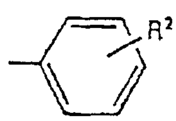

($R^2$ is a hydrogen atom, alkyl group having 1-6 carbon atoms or alkoxy group having 1-6 carbon atoms), naphthyl radical, pyridyl radical, furyl radical, thienyl radical, methylphenylsulfonyl radical, $-COCF_3$ radical, $-COOR^1$ group, ($R^1$ is an alkyl group having 1-6 carbon atoms) or $-COCH(NH_2)R^3$ group, ($R^3$ is a hydrogen atom or an alkyl group having 1-6 carbon atoms), but W and Z do not represent same meaning of hydrogen atom, or a salt thereof, for the manufacture of a medicament for a treatment wherein the Maillard reaction is inhibited.

**3.** The use of a hydantoin derivative as claimed in Claim 2, wherein said hydantoin derivative is selected from the group consisting of

(A) (2S,4S)-6-Fluoro-2',5'-dioxospiro[chroman-4,4'-imidazolidine]-2-carbohydrazide,
(B) (2R,4S)-6-Fluoro-2',5'-dioxospiro[chroman-4,4'-imidazolidine]-2-carbohydrazide,
(C) (2S,4R)-6-Fluoro-2',5'-dioxospiro[chroman-4,4'-imidazolidine]-2-carbohydrazide,
(D) (2R,4R)-6-Fluoro-2',5'-dioxospiro[chroman-4,4'-imidazolidine]-2-carbohydrazide,
(E) (2S, 4S)-6-Fluoro-N'-methyl-2',5'-dioxospiro[chroman-4,4'-imidazolidine]-2-carbohydrazide
(F) (2S, 4S)-6-Fluoro-2',5'-dioxo-N'-phenylspiro[chroman-4,4'-imidazolidine]-2-carbohydrazide
(G) (2S, 4S)-N'-(4-Chlorophenyl)-6-fluoro-2',5'-dioxospiro[chroman-4,4'-imidazolidine]-2-carbohydrazide
(H) (2S, 4S)-6-Fluoro-N'-(4-methoxyphenyl)-2',5'-dioxospiro[chroman-4,4'-imidazolidine]-2-carbohydrazide
(I) (2S, 4S)-6-Fluoro-N'-(1-naphthyl)-2',5'-dioxospiro[chroman-4,4'-imidazolidine]-2-carbohydrazide

(J) (2S, 4S)-N'-Acetyl-6-fluoro-2',5-dioxospiro[chroman-4,4'-imidazolidine]-2-carbohydrazide
(K) (2S, 4S)-6-Fluoro-N'-(4-methylphenylsulfonyl)-2',5'-dioxospiro[chroman-4,4'-imidazolidine]-2-carbohydrazide
(L) (2S, 4S)-N'-tert-Butoxycarbonyl-6-fluoro-2',5'-dioxospiro[chroman-4,4'-imidazolidine]-2-carbohydrazide
(M) (2S, 4S)-N'-L-Alanyl-6-fluoro-2',5'-dioxospiro[chroman-4,4'-imidazolidine]-2-carbohydrazide
(N) (2S, 4S)-6-Fluoro-2',5'-dioxo-N'-trifluoroacetylspiro[chroman-4,4'-imidazolidine]-2-carbohydrazide
(O) (2S,4S)-1'-Amino-6-fluoro-2',5'-dioxospiro[chroman-4,4'-imidazolidine]-2-carbohydrazide,
(P) (2S,4S-)-1'-Amino-6-fluoro-2',5'-dioxospiro[chroman-4,4'-imidazolidine]-2-carboxamide, and
(Q) a salt of the above compounds.

## Patentansprüche

1. Hydantoin-Derivat, ausgewählt aus der Gruppe bestehend aus

(A) (2R,4S)-6-Fluor-2',5'-dioxospiro[chroman-4,4'-imidazolin]-2-carbohydrazid,
(B) (2S,4R)-6-Fluor-2',5'-dioxospiro[chroman-4,4'-imidazolin]-2-carbohydrazid,
(C) (2R,4R)-6-Fluor-2',5'-dioxospiro[chroman-4,4'-imidazolin]-2-carbohydrazid,
(D) (2S,4S)-N'-Acetyl-6-fluor-2',5'-dioxospiro[chroman-4,4'-imidazolin]-2-carbohydrazid,
(E) (2S,4S)-6-Fluor-N'-(4-methylphenylsulfonyl)-2',5'-dioxospiro[chroman-4,4'-imidazolin]-2-carbohydrazid,
(F) (2S,4S)-N'-tert-Butoxycarbonyl-6-fluor-2',5'-dioxospiro[chroman-4,4'-imidazolin]-2-carbohydrazid,
(G) (2S,4S)-N'-L-Alanyl-6-fluor-2',5'-dioxospiro[chroman-4,4'-imidazolin]-2-carbohydrazid,
(H) (2S,4S)-6-Fluor-2',5'-dioxo-N'-trifluoracetylspiro[chroman-4,4'-imidazolin]-2-carbohydrazid,
Q) (2S,4S)-1'-Amino-6-fluor-2',5'-dioxospiro[chroman-4,4'-imidazolin]-2-carbohydrazid und
(J) (2S,4S)-1'-Amino-6-fluor-2',5'-dioxospiro[chroman-4,4'-imidazolin]-2-carboxamid

oder ein Salz der Derivate.

2. Verwendung eines Hydantoin-Derivates der Formel

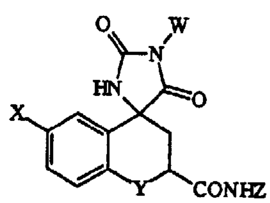

worin W ein Wasserstoffatom oder ein Aminorest ist, X ein Wasserstoffatom, Halogenatom, eine Alkylgruppe mit 1 bis 6 Kohlenstoffatomen oder eine Alkoxygruppe mit 1 bis 6 Kohlenstoffatomen ist, Y ein Sauerstoffatom oder Schwefelatom ist und Z ein Wasserstoffatom, eine -NHR-Gruppe, in der R ein Wasserstoffatom, eine Alkylgruppe mit 1 bis 6 Kohlenstoffatomen, eine Alkanoylgruppe mit 1 bis 6 Kohlenstoffatomen, eine Gruppe der Formel

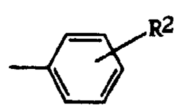

($R^2$ ist ein Wasserstoffatom, eine Alkylgruppe mit 1 bis 6 Kohlenstoffatomen oder eine Alkoxygruppe mit 1 bis 6 Kohlenstoffatomen), ein Naphthylrest, Pyridylrest, Furylrest, Thienylrest, Methylphenylsulfonylrest, $-COCF_3$-Rest, eine $-COOR^1$-Gruppe ($R^1$ ist eine Alkylgruppe mit 1 bis 6 Kohlenstoffatomen) oder eine $-COCH(NH_2)R^3$-Gruppe ist ($R^3$ ist ein Wasserstoffatom oder eine Alkylgruppe mit 1 bis 6 Kohlenstoffatomen), doch repräsentieren W und Z nicht die gleiche Bedeutung Wasserstoffatom, oder ein Salz davon, zur Herstellung eines Medikamentes für eine Behandlung, bei der die Maillard-Reaktion gehemmt wird.

3. Verwendung eines Hydantoin-Derivates nach Anspruch 2, worin das Hydantoin-Derivat ausgewählt ist aus der Gruppe bestehend aus

(A) (2S,4S)-6-Fluor-2',5',-dioxospiro[chroman-4,4'-imidazolin]-2-carbohydrazid,
(B) (2R,4S)-6-Fluor-2',5'-dioxospiro[chroman-4,4'-imidazolin]-2-carbohydrazid,
(C) (2S,4R)-6-Fluor-2',5'-dioxospiro[chroman-4,4'-imidazolin]-2-carbohydrazid,
(D) (2R,4R)-6-Fluor-2',5'-dioxospiro[chroman-4,4'-imidazolin]-2-carbohydrazid,
(E) (2S,4S)-6-Fluor-N'-methyl-2',5'-dioxospiro[chroman-4,4'-imidazolin]-2-carbohydrazid,
(F) (2S,4S)-6-Fluor-2',5'-dioxo-N'-phenylspiro[chroman-4,4'-imidazolin]-2-carbohydrazid,
(G) (2S,4S)-N'-(4-Chlorphenyl)-6-fluor-2',5'-dioxospiro[chroman-4,4'-imidazolin]-2-carbohydrazid,
(H) (2S,4S)-6-Fluor-N'-(4-methoxyphenyl)-2',5'-dioxospiro[chroman-4,4'-imidazolin]-2-carbohydrazid,
(I) (2S,4S)-6-Fluor-N'-(1-naphthyl)-2',5'-dioxospiro[chroman-4,4'-imidazolin]-2-carbohydrazid,
(J) (2S,4S)-N'-Acetyl-6-fluor-2',5'-dioxospiro[chroman-4,4'-imidazolin]-2-carbohydrazid,
(K) (2S,4S)-6-Fluor-N'-(4-methylphenysulfonyl)-2',5'-dioxospiro[chroman-4,4'-imidazolin]-2-carbohydrazid,
(L) (2S,4S)-N'-tert-Butoxycarbonyl-6-fluor-2',5'-dioxospiro[chroman-4,4'-imidazolin]-2-carbohydrazid,
(M) (2S,4S)-N'-L-Alanyl-6-fluor-2',5'-dioxospiro[chroman-4,4'-imidazolin]-2-carbohydrazid,
(N) (2S,4S)-6-Fluor-2',5'-dioxo-N'-trifluoracetylspiro[chroman-4,4'-imidazolin]-2-carbohydrazid,
(O) (2S,4S)-1'-Amino-6-fluor-2',5'-dioxospiro[chroman-4,4'-imidazolin]-2-carbohydrazid,
(P) (2S,4S)-1'-Amino-6-fluor-2',5'-dioxospiro[chroman-4,4'-imidazolin]-2-carboxamid und
(Q) ein Salz von den oberen Verbindungen.

**Revendications**

1. Dérivé d'hydantoïne, choisi dans le groupe consistant en :

(A) (2R,4S)-6-fluoro-2',5'-dioxospiro[chromanne-4,4'-imidazolidine]-2-carbohydrazide,
(B) (2S,4R)-6-fluoro-2',5'-dioxospiro[chromanne-4,4'-imidazolidine]-2-carbohydrazide,
(C) (2R,4R)-6-fluoro-2',5'-dioxospiro[chromanne-4,4'-imidazolidine]-2-carbohydrazide,
(D) (2S,4S)-N'-acétyl-6-fluoro-2',5'-dioxospiro[chromanne-4,4'-imidazolidine]-2-carbohydrazide,
(E) (2S,4S)-6-fluoro-N'-(4-méthylphénylsulfonyl)-2',5'-dioxospiro[chromanne-4,4'-imidazolidine]-2-carbohydrazide,
(F) (2S,4S)-N'-tert-butoxycarbonyl-6-fluoro-2',5'-dioxospiro[chromanne-4,4'-imidazolidine]-2-carbohydrazide,
(G) (2S,4S)-N'-L-alanyl-6-fluoro-2',5'-dioxospiro[chromanne-4,4'-imidazolidine]-2-carbohydrazide,
(H) (2S,4S)-6-fluoro-2',5'-dioxo-N'-trifluoroacétylspiro[chromanne-4,4'-imidazolidine]-2-carbohydrazide,
(I) (2S,4S)-1'-amino-6-fluoro-2',5'-dioxospiro[chromanne-4,4'-imidazolidine]-2-carbohydrazide, et
(J) (2S,4S)-1'-amino-6-fluoro-2',5'-dioxospiro[chromanne-4,4'-imidazolidine]-2-carboxamide,

ou un sel des dérivés.

2. Utilisation d'un dérivé d'hydantoïne de formule

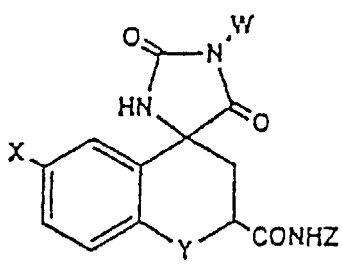

(II)

dans laquelle W est un atome d'hydrogène ou un radical amino ; X est un atome d'hydrogène, un atome d'halogène, un groupe alkyle ayant 1-6 atomes de carbone ou un groupe alcoxy ayant 1-6 atomes de carbone ; Y est un atome d'oxygène ou un atome de soufre ; et Z est un atome d'hydrogène, un groupe -NHR dans lequel R est un atome d'hydrogène, un groupe alkyle ayant 1-6 atomes de carbone, un groupe alcanoyle ayant 1-6 atomes de carbone, un groupe

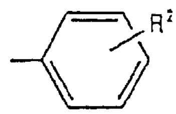

($R^2$ est un atome d'hydrogène, un groupe alkyle ayant 1-6 atomes de carbone ou un groupe alcoxy ayant 1-6 atomes de carbone), le radical naphtyle, le radical pyridyle, le radical furyle, le radical thiényle, le radical méthylphénylsulfonyle, le radical-$COCF_3$, un groupe -$COOR^1$ ($R^1$ est un groupe alkyle ayant 1-6 atomes de carbone) ou un groupe -$COCH(NH_2)R^3$ ($R^3$ est un atome d'hydrogène ou un groupe alkyle ayant 1-6 atomes de carbone), mais W et Z ne représentent pas simultanément un atome d'hydrogène, ou d'un de ses sels, pour la préparation d'un médicament destiné à un traitement dans lequel la réaction de Maillard est inhibée.

**3.** Utilisation d'un dérivé d'hydantoïne selon la revendication 2, dans lequel ledit dérivé d'hydantoïne est choisi dans le groupe consistant en :

(A) (2S,4S)-6-fluoro-2',5'-dioxospiro[chromanne-4,4'-imidazolidine]-2-carbohydrazide,
(B) (2R,4S)-6-fluoro-2',5'-dioxospiro[chromanne-4,4'-imidazolidine]-2-carbohydrazide,
(C) (2S,4R)-6-fluoro-2',5'-dioxospiro[chromanne-4,4'-imidazolidine]-2-carbohydrazide,
(D) (2R,4R)-6-fluoro-2',5'-dioxospiro[chromanne-4,4'-imidazolidine]-2-carbohydrazide,
(E) (2S,4S)-6-fluoro-N'-méthyl-2',5'-dioxospiro[chromanne-4,4'-imidazolidine]-2-carbohydrazide,
(F) (2S,4S)-6-fluoro-2',5'-dioxo-N'-phénylspiro[chromanne-4,4'-imidazolidine]-2-carbohydrazide,
(G) (2S,4S)-N'-(4-chlorophényl)-6-fluoro-2',5'-dioxospiro[chromanne-4,4'-imidazolidine]-2-carbohydrazide,
(H) (2S,4S)-6-fluoro-N'-(4-méthoxyphényl)-2',5'-dioxospiro[chromanne-4,4'-imidazolidine]-2-carbohydrazide,
(I) (2S,4S)-6-fluoro-N'-(1-naphtyl)-2',5'-dioxospiro[chromanne-4,4'-imidazolidine]-2-carbohydrazide,
(J) (2S,4S)-N'-acétyl-6-fluoro-2',5'-dioxospiro[chromanne-4,4'-imidazolidine]-2-carbohydrazide,
(K) (2S,4S)-6-fluoro-N'-(4-méthylphénylsulfonyl)-2',5'-dioxospiro[chromanne-4,4'-imidazolidine]-2-carbohydrazide,
(L) (2S,4S)-N'-tert-butoxycarbonyl-6-fluoro-2',5'-dioxospiro[chromanne-4,4'-imidazolidine]-2-carbohydrazide,
(M) (2S,4S)-N'-L-alanyl-6-fluoro-2',5'-dioxospiro[chromanne-4,4'-imidazolidine]-2-carbohydrazide;
(N) (2S,4S)-6-fluoro-2',5'-N'-trifluoroacétylspiro[chromanne-4,4'-imidazolidine]-2-carbohydrazide,
(O) (2S,4S)-1'-amino-6-fluoro-2',5'-dioxospiro[chromanne-4,4'-imidazolidine]-2-carbohydrazide,
(P) (2S,4S)-1'-amine-6-fluoro-2',5'-dioxospiro[chromanne-4,4'-imidazolidine]-2-carboxamide, et
(Q) un sel des composés ci-dessus.